# EUROPEAN PATENT APPLICATION

(11) **EP 1 752 087 A2**
(43) Date of publication of application: **14.02.2007**
(21) Application number: 06016134.6
(22) Date of filing: 02.08.2006
(51) Int. Cl.: A61B 5/022

(54) **Blood pressure monitor capable of managing measurement value of multiple users**

(30) Priority: 11.08.2005 JP 2005233346
(71) Applicant: Omron Healthcare Co., Ltd., Kyoto 615-0084 (JP)
(72) Inventor: Kishimoto, Hiroshi, Kyoto-shi Kyoto 615-0084 (JP); Sawanoi, Yukiya, Kyoto-shi Kyoto 615-0084 (JP); Matsumura, Naomi, Kyoto-shi Kyoto 615-0084 (JP); Tanaka, Takahide, Kyoto-shi Kyoto 615-0084 (JP)
(74) Representative: Kilian, Helmut

(57) **Abstract**

A blood pressure monitor (1) includes a switch (11A, 11B) for designating a user who is a subject. The switch (11A, 11B) for designating a user also serves as a power switch. When this switch is pressed as the first step for blood pressure measurement, the blood pressure monitor (1) is turned on. The blood pressure monitor (1) does not operate even when a switch (12) for blood pressure measurement operation or a switch (14) for display of recorded past measurement values is pressed before the switch (11A, 11B) for designating a user is pressed.

## Description

### BACKGROUND OF THE INVENTION

### Field of the Invention

The present invention relates to a blood pressure monitor, and more particularly to a blood pressure monitor for shared use by a plurality of users.

### Description of the Background Art

In recent years, many people suffer from lifestyle diseases caused by hypertension. A blood pressure level is used as one index for health management for preventing/overcoming such lifestyle diseases. Accordingly, blood pressure management is an important factor.

Blood pressure may be measured in a hospital, while it may be measured also at home in recent days. In such a case, it is possible that one household owns one blood pressure monitor for shared use by a plurality of users (for example, family members) for blood pressure measurement.

In managing blood pressure measurement record values of a plurality of users in one blood pressure monitor, it is essential to avoid mix-up of the record values. As a method for managing blood pressure values and a blood pressure monitor for that purpose, the following techniques are available.
(1) The measurement values are recorded in a magnetic card owned by an individual. The blood pressure monitor reads the record value from the magnetic card and displays trend graph (Japanese Patent Laying-Open No. 05-137698).
(2) After the blood pressure is measured, whether the measurement value is to be recorded or not is selected. If the measurement value is to be recorded, a storage area (for example, two persons × 14 times) is selected (Japanese Utility Model Registration No. 3020497).

The following is also one of other references:
A&D Co., LTD., "Digital Blood Pressure Monitor Model UA-774," [online], [searched on July 26, 2005], Internet <http://www.aandd.co.jp/eadhome/html/products/medical/consumer/ua774.html>.

According to the conventional configuration as configuration (1) above, however, a magnetic card or the like for recording individual data including a measurement result is required, and the price of a blood pressure monitor tends to be higher than that for a general household.

Meanwhile, if selection as to whether or not the measurement value is to be recorded or selection of the storage area is permitted as in configuration (2) above, a user records solely data favorable to him/herself, and accurate blood pressure management becomes impossible. In addition, if every measurement value is recorded without fail but selection of the storage area is not proper, the measurement values are mixed up.

### SUMMARY OF THE INVENTION

From the foregoing, the present invention was made paying attention to the above-described problems, and an object of the present invention is to provide a blood pressure monitor preventing mix-up of measurement values when used by a plurality of users as well as storing measured data.

In order to achieve the object above, according to one aspect of the present invention, a blood pressure monitor includes: a first manipulation portion for selecting a storage area corresponding to a subject; a second manipulation portion for manipulation for operating the blood pressure monitor; and a control unit prohibiting manipulation of the second manipulation portion until selection in the first manipulation portion is made.

Preferably, the blood pressure monitor further includes a measurement unit measuring blood pressure of the subject and storing a result of measurement in the storage area corresponding to the subject, and the second manipulation portion includes a manipulation portion for manipulation for operating the measurement unit.

As the blood pressure monitor according to the present invention is configured in such a manner, the user selects the storage area prior to use without fail, and the result of measurement is recorded in such a storage area after measurement. In addition, as the storage area corresponding to the user who is the subject is designated, measurement record values of a plurality of users are not mixed up.

Preferably, the first manipulation portion also serves as a manipulation portion for turning on the blood pressure monitor. With such a configuration, initialization processing is performed after the storage area is set, without coming to naught, and more efficient processing in the blood pressure monitor can be achieved.

Preferably, the blood pressure monitor further includes a display unit displaying a result of measurement, and at least display on the display unit is started by manipulation in the first manipulation portion.

Preferably, if the first manipulation portion is manipulated after the storage area is selected through the first manipulation portion and before a series of operations of the blood pressure monitor with respect to the subject ends, the control unit ends the operations of the blood pressure monitor.

In addition, preferably, the second manipulation portion serves as a manipulation portion causing the blood pressure monitor to operate with respect to the subject selected in the first manipulation portion.

As described above, as compared with a configuration in which a switch corresponding to a function is arranged for each user, the number of switches can be reduced, and interface graphic configuration can be simplified, which improves operability for the user. For example, even an elderly user, an optically challenged user, or a physically challenged user in his/her hand manipulates the blood pressure monitor can manipulate the blood pressure monitor smoothly, without difficulty.

The foregoing and other objects, features, aspects and advantages of the present invention will become more apparent from the following detailed description of the present invention when taken in conjunction with the accompanying drawings.

### BRIEF DESCRIPTION OF THE DRAWINGS

Fig. 1 illustrates a specific example of appearance of a blood pressure monitor 1.
Fig. 2 is a block diagram showing a specific example of a configuration of blood pressure monitor 1.
Fig. 3 illustrates a first specific example of a configuration of a memory 9.
Fig. 4 illustrates a second specific example of a configuration of memory 9.
Fig. 5 is a block diagram showing a specific example of a functional configuration of blood pressure monitor 1.
Fig. 6 is a flowchart showing processing in blood pressure monitor 1.

### DESCRIPTION OF THE PREFERRED EMBODIMENTS

An embodiment of the present invention will be described hereinafter with reference to the drawings. In the description below, the same parts and elements have the same reference characters allotted, and their label and function are also identical.

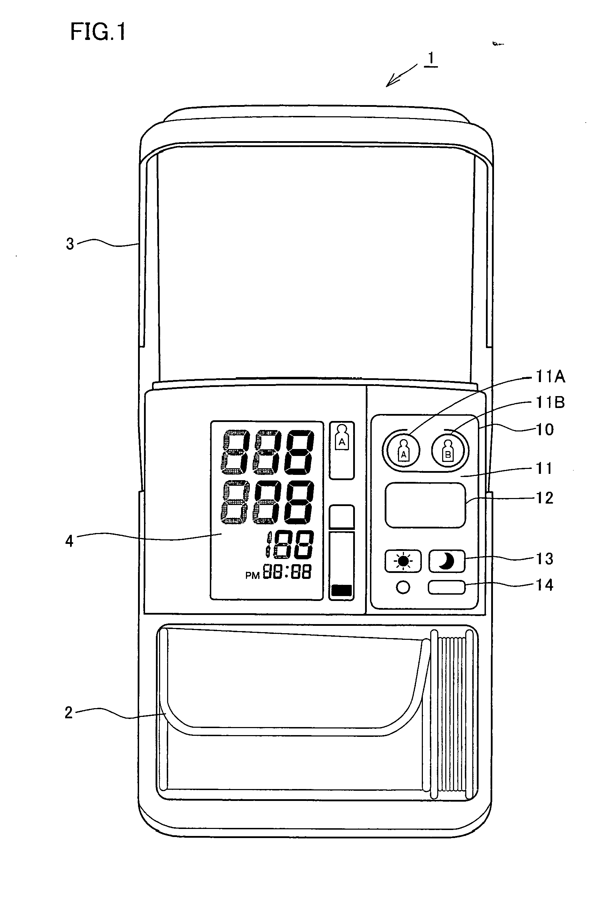

Fig. 1 is a front view of a blood pressure monitor 1 when a cover 3 covering entire blood pressure monitor 1 is opened (at the time of start of use).

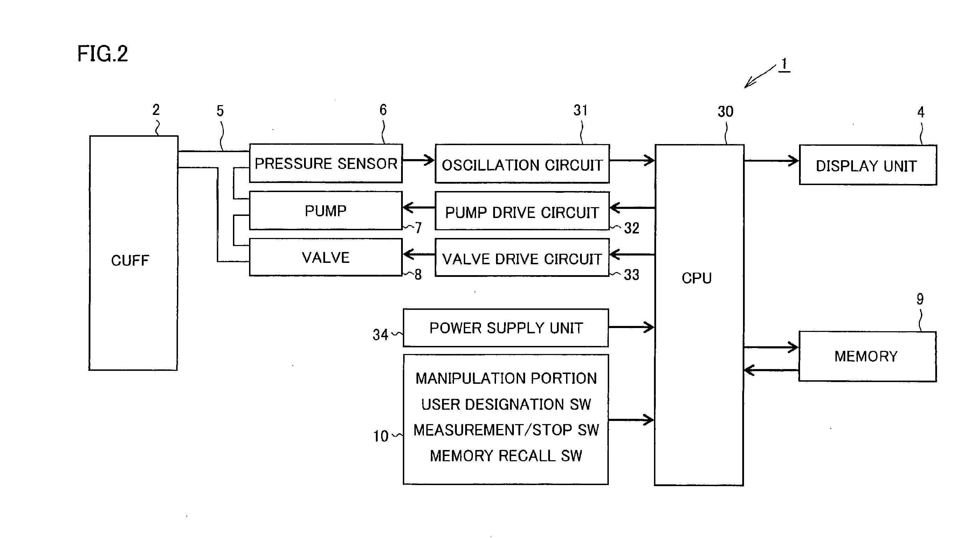

Referring to Fig. 1, a cuff (arm band) 2 is connected to a main unit of blood pressure monitor 1 according to the present embodiment through an air hose 5 (as Fig. 1 shows a state in which cuff 2 is stored, air hose 5 is not shown in Fig. 1). A patient, a doctor, or a user at home places cuff 2 on any limb, such as upper arm or wrist of a subject, so as to measure blood pressure. Measurement of blood pressure is controlled by a CPU (Central Processing Unit) 30 (see Fig. 2) included in blood pressure monitor 1.

In addition, referring to Fig. 1, a manipulation portion 10 implemented by a group of switches for various types of manipulation of blood pressure monitor 1 and a display unit 4 implemented by a liquid crystal panel or the like and displaying a result of measurement or the like are provided on the surface of blood pressure monitor 1 main unit. Manipulation portion 10 includes a switch 11 for designating a user who conducts measurement (or causing a past measurement value to be displayed), a switch 12 for instructing start/stop of measurement of blood pressure, a switch 13 for input of a condition at the time of measurement of blood pressure, such as a switch 13A for input of measurement time as morning and a switch 13B for input of measurement time as evening, and a switch 14 for displaying a past measurement value. If blood pressure monitor 1 attains a function other than measurement of blood pressure and display of the past measurement value, manipulation portion 10 may include a switch corresponding to that function.

Fig. 2 shows a specific example of a configuration of blood pressure monitor 1 when blood pressure measurement adopting an oscillometric blood pressure measurement principle is carried out. This blood pressure measurement principle represents merely one specific example, and the present invention is not limited thereto.

Referring to Fig. 2, blood pressure monitor 1 includes a pressure sensor 6, pump 7 and a valve 8 for operation for blood pressure measurement using cuff 2, as well as an oscillation circuit 31, a pump drive circuit 32 and a valve drive circuit 33 that are circuits driving these components respectively. Oscillation circuit 31, pump drive circuit 32 and valve drive circuit 33 are connected to CPU 30.

CPU 30 is supplied with power from a power supply unit 34, outputs a control signal to pump drive circuit 32 and valve drive circuit 33 in response to a manipulation signal from manipulation portion 10, and controls an operation for measurement of blood pressure. In addition, CPU 30 receives a sensor signal from oscillation circuit 31, performs prescribed operational processing, and obtains a measurement result.

CPU 30 is further connected to display unit 4 and memory 9, outputs a control signal to display unit 4, and causes the obtained measurement result to be displayed, or stored in memory 9. Moreover, in response to a manipulation signal from manipulation portion 10, CPU 30 reads the stored measurement value from memory 9 and causes display unit 4 to display the measurement value.

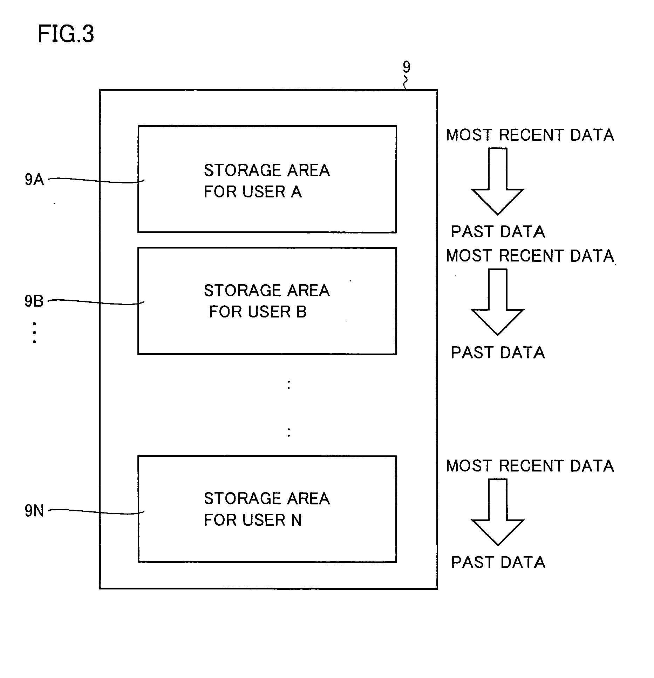

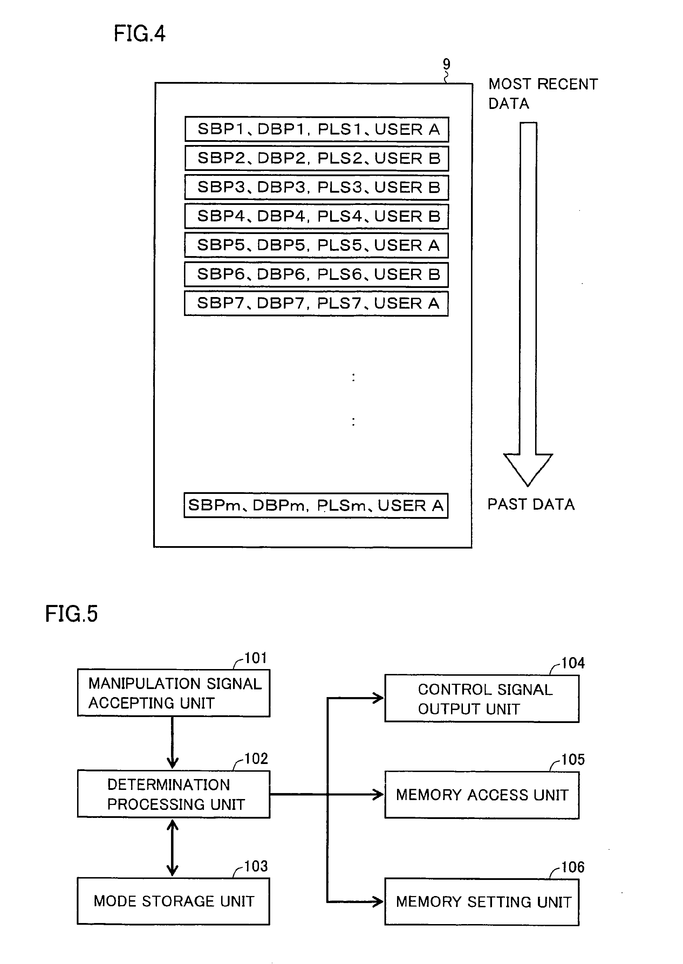

Memory 9 stores the measurement value in blood pressure monitor 1 for each user who is the subject, along with a program executed by CPU 30. Two configurations (Figs. 3 and 4) below specifically represent exemplary configurations of memory 9.

Referring to Fig. 3, as a first specific example of the configuration of memory 9, memory 9 is configured to include storage areas 9A, 9B, ... 9N for each user (registered as) using blood pressure monitor 1. The measurement value of the user is stored in the storage area for each user, for example, in a reverse chronological (or chronological) order.

Referring to Fig. 4, as a second specific example of the configuration of memory 9, memory 9 stores SBP (Systolic Blood Pressure: maximal blood pressure), DBP (Diastolic Blood Pressure: minimal blood pressure) and PLS (Pulse Rate) that represent measurement values obtained in one measurement, in association with identification data for identifying a user who is the subject, for example, in a reverse chronological (or chronological) order.

In the present embodiment, blood pressure monitor 1 main unit is automatically turned on in accordance with an operation to open cover 3. Here, turn-on of blood pressure monitor 1 main unit refers to such a state that power is supplied from power supply unit 34 to CPU 30, CPU 30 functions at least in a low power state, and CPU 30 can receive a manipulation signal from switch 11.

In the present embodiment, preferably, switch 11 for designating a user also serves as the "power switch", and more preferably, switch 11 is labeled as the "power switch" on blood pressure monitor 1 main unit.

It is noted that the "power switch" in the present embodiment refers to a switch for turning on blood pressure monitor 1 in order to allow subsequent manipulation. More specifically, the "power switch" refers to a switch for executing processing to start at least display on display unit 4, in order to allow subsequent manipulation. In addition, if CPU 30 functions in a low power state during a period from turn-on of blood pressure monitor 1 main unit until pressing of the "power switch", the "power switch" refers to a switch for transition of an operation state of CPU 30 from the low power state to a normal power state. Moreover, preferably, by pressing the "power switch", initialization of each circuit 31 to 33 for allowing a measurement operation and preparation for measurement such as measurement of an atmospheric pressure is started in CPU 30. Such processing, however, may be started before pressing of the "power switch" (for example, at the timing of turn-on of blood pressure monitor 1 main unit).

Preferably, the number of switches 11 corresponds to the predetermined number of users as shown in Fig. 1, and switch 11 is configured such that the user can be designated by pressing switch 11 corresponding to a user who is the subj ect. Specifically, switches 11 shown in Fig. 1 include a switch 11A for designating user A who is a first user and a switch 11B for designating user B who is a second user. Switches for manipulating blood pressure monitor 1 other than switch 11 are provided in correspondence with each manipulation (in common to the users) as shown in Fig. 1.

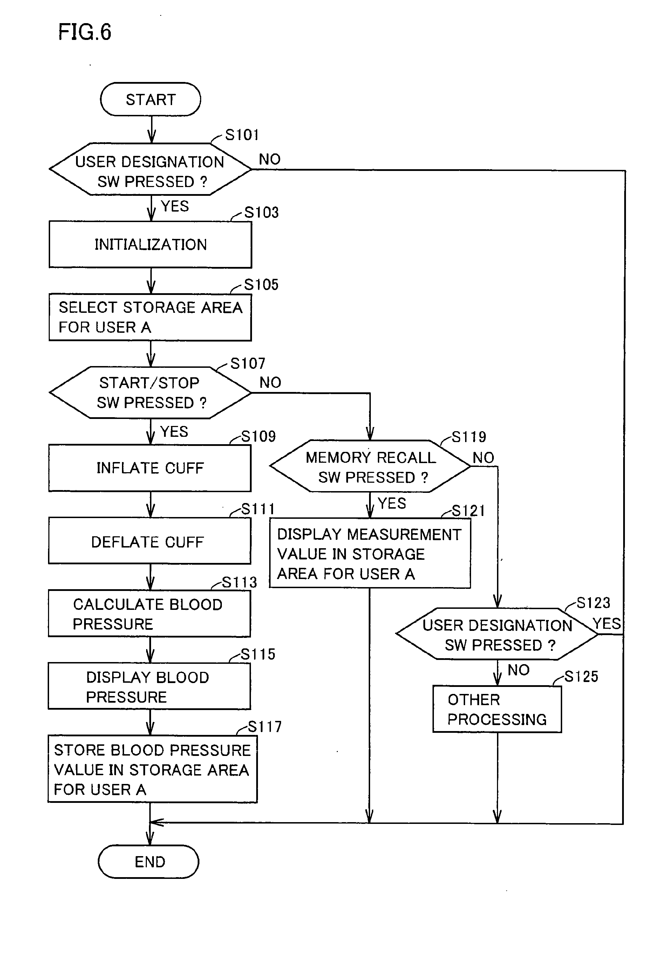

Each function of blood pressure monitor 1 shown in Fig. 5 is attained in CPU 30 as a result of execution by CPU 30 in Fig. 2 of a program stored in memory 9. Alternatively, some of these functions may be attained by using a hardware configuration shown in Fig. 2.

Referring to Fig. 5, the function of blood pressure monitor 1 is attained by a manipulation signal accepting unit 101 accepting a manipulation signal from manipulation portion 10, a determination processing unit 102, a mode storage unit 103, a control signal output unit 104 outputting control signals to pump drive circuit 32, valve drive circuit 33 and display unit 4, a memory access unit 105 accessing memory 9 and storing/reading necessary data, and a memory setting unit 106 setting a storage area used for processing when the memory is configured to include the storage area for each user shown in Fig. 3.

Upon receiving from manipulation signal accepting unit 101, the manipulation signal accepted through manipulation portion 10, determination processing unit 102 performs the following processing, referring to mode storage unit 103.

If the manipulation signal received at the beginning of a series of manipulations is the manipulation signal designating a user who is the subject through manipulation of switch 11, that is, if the designated user has not been stored in mode storage unit 103 and if the manipulation signal generated through manipulation of switch 11 is accepted, determination processing unit 102 stores the designated user in mode storage unit 103. In addition, for display necessary for subsequent manipulation, determination processing unit 102 outputs an instruction signal to control signal output unit 104, so as to output a control signal to display unit 4. Moreover, determination processing unit 102 outputs an instruction signal to control signal output unit 104, so as to output a control signal for executing initialization processing for allowing the measurement operation to pump drive circuit 32 and/or valve drive circuit 33. Meanwhile, when the memory is configured to include the storage area for each user shown in Fig. 3, determination processing unit 102 outputs an instruction signal to memory setting unit 106, such that a storage area associated with the designated user is set as the storage area used in subsequent processing.

If the manipulation signal received while the user who is the subject has been designated is the manipulation signal generated as a result of manipulation of switch 12, switch 13A, switch 13B, or switch 14, that is, if the designated user is stored in mode storage unit 103 and if the manipulation signal generated as a result of manipulation of switch 12, switch 13A, switch 13B, or switch 14 is accepted, determination processing unit 102 outputs an instruction signal to control signal output unit 104, so as to output a control signal to pump drive circuit 32, valve drive circuit 33 and/or display unit 4 so that the processing in accordance with the manipulation signal is executed. Alternatively, determination processing unit 102 outputs an instruction signal to memory access unit 105 along with information specifying the currently selected user, so that prescribed data is acquired from memory 9.

If the manipulation signal received at the beginning of a series of manipulations is the manipulation signal other than that generated as a result of manipulation of switch 11, that is, if the designated user has not been stored in mode storage unit 103 and if the manipulation signal generated as a result of manipulation of a switch other than switch 11 is accepted, determination processing unit 102 outputs an instruction signal neither to control signal output unit 104 nor to memory access unit 105. That is, if switch 12 or the like is pressed before user designation through switch 11, determination processing unit 102 does not perform the measurement operation or the like, but prohibits manipulation of a switch other than switch 11, such as switch 12.

If the manipulation signal received while the user who is the subject has been designated is the manipulation signal designating the user who is the subject generated as a result of manipulation of switch 11, that is, if the designated user has been stored in mode storage unit 103 and if the manipulation signal generated as a result of manipulation for selecting the same user again or the manipulation signal generated as a result of manipulation for selecting other user is accepted, determination processing unit 102 outputs an instruction signal to control signal output unit 104, so as to output a control signal to display unit 4 so that display necessary for subsequent manipulation is not permitted, thereby disabling subsequent manipulation. If the blood pressure monitor is performing the measurement operation, determination processing unit 102 outputs an instruction signal to control signal output unit 104, so as to output a control signal to pump drive circuit 32 and/or valve drive circuit 33 so that the operation ends.

The processing shown in the flowchart in Fig. 6 is executed as a result of reading and execution of a program stored in memory 9 by CPU 30 for attaining each function shown in Fig. 5.

Referring to Fig. 6, initially, when any user is selected by pressing switch 11 (YES in step S101), blood pressure monitor 1 main unit is turned on, and initialization processing for allowing the subsequent measurement operation and preparation for measurement such as measurement of an atmospheric pressure are performed (step S103). In addition, display on display unit 4 is turned on, in order to enable subsequent processing. Moreover, the storage area for the user (for example, user A) designated in step S101 is selected and set as the storage area used in the subsequent processing, out of the storage areas in memory 9 (step S105).

Successively, when switch 12 is pressed (YES in step S107), the measurement operation is started (steps S 109 to S117). That is, pump 7 is driven by pump drive circuit 32 to inflate cuff 2 (step S109). Successively, after valve 8 is driven by valve drive circuit 33 and switching is made, pump 7 is driven by pump drive circuit 32 to deflate cuff 2 (step S111). The pressure in cuff 2 and air hose 5 during pressure application and reduction is measured by pressure sensor 6, and the measured pressure signal is transmitted to CPU 30 through oscillation circuit 31.

CPU 30 calculates the blood pressure based on an inner pressure measurement value from pressure sensor 6 (step S113), and causes display unit 4 to display the measurement result (step S115). In addition, CPU 30 causes memory 9 to store the measurement value in the storage area for the user selected in step S105 (step S 117).

When the measurement operation above ends, a series of processes ends. When the process ends, the power supplied to CPU 30 may automatically be cut off, transition of the power state of CPU 30 may be made to the low power state, or display on display unit 4 may automatically be turned off so as not to allow subsequent manipulation.

If a switch other than switch 11 is pressed at the time of start of the processing above (NO in step S101), the processing ends without performing the processing in step S 103 or later. Manipulation of a switch other than switch 11, such as switch 12, without designating a user in advance through manipulation of switch 11 is thus prohibited.

In addition, if switch 14 is pressed after selection and setting of the storage area for the designated user in memory 9 in step S105 (NO in step S107 and YES in S 119), the measurement value is recalled from the storage area selected in step S105 and displayed on display unit 4 (step S121).

Alternatively, if a switch corresponding to other function, other than switch 12 and switch 14, is pressed (NO in steps S107, S119, S 123), the processing corresponding to that switch is performed (step S125).

If switch 11 is again pressed after switch 11 is once pressed for selecting any user in step S101 above (NO in steps S107, S119 and YES in S123), a series of processes ends without performing subsequent processing such as measurement operation or the like.

Blood pressure monitor 1 according to the present embodiment performs the processing as described above, so that subsequent manipulation is not allowed in blood pressure monitor 1 unless selection of the storage area is made. Therefore, the user never fails to select the storage area prior to use, and the measurement result is necessarily recorded in that storage area after measurement. In addition, as the storage area corresponding to the user who is the subject is designated, the measurement record values of a plurality of users are not mixed up.

In addition, as described above, in the present embodiment, switch 11 for designating a user is presented also as the "power button". Therefore, the user performs manipulation for designating the storage area without fail before manipulation for measurement.

Moreover, the initialization processing is performed after setting of the storage area, without coming to naught. Therefore, more efficient processing in blood pressure monitor 1 can be achieved.

In blood pressure monitor 1 according to the present embodiment, the user which is the subject is designated through switch 11A or switch 11B and thereafter subsequent manipulation is performed. Therefore, the number of switches 11 alone should correspond to the number of users, while the switches corresponding to the functions such as switches 12 and 14 can be used in common to the users. Accordingly, as compared with a configuration in which a switch corresponding to a function is arranged for each user, the number of switches can be reduced, and interface graphic configuration can be simplified, which improves operability for the user. For example, even an elderly user, an optically challenged user, or a physically challenged user in his/her hand can manipulate the blood pressure monitor smoothly, without difficulty.

Moreover, the configuration in which the number of switches is reduced or the configuration in which the measurement record values for each user are managed without using a magnetic card or the like as described above can achieve lower cost.

Although the present invention has been described and illustrated in detail, it is clearly understood that the same is by way of illustration and example only and is not to be taken by way of limitation, the spirit and scope of the present invention being limited only by the terms of the appended claims.

## Claims

1. A blood pressure monitor, comprising:
a first manipulation portion (11) for selecting a storage area corresponding to a subject;
a second manipulation portion (12, 13, 14) for manipulation for operating the blood pressure monitor; and
a control unit (30) prohibiting manipulation of said second manipulation portion until selection in said first manipulation portion is made.

2. The blood pressure monitor according to claim 1, further comprising a measurement unit (2, 5, 6, 7, 8, 30, 31, 32, 33) measuring blood pressure of said subject and storing a result of measurement in said storage area; wherein
said second manipulation portion includes a manipulation portion (12) for manipulation for operating said measurement unit.

3. The blood pressure monitor according to claim 1, wherein
said first manipulation portion also serves as a manipulation portion for turning on the blood pressure monitor.

4. The blood pressure monitor according to claim 3, further comprising a display unit (4) displaying a result of measurement; wherein
at least display on said display unit is started by manipulation in said first manipulation portion.

5. The blood pressure monitor according to claim 1, wherein
if said first manipulation portion is manipulated after said storage area is selected through said first manipulation portion and before a series of operations of the blood pressure monitor with respect to said subject ends, said control unit ends said operations without performing an operation subsequent to said operations of the blood pressure monitor.

6. The blood pressure monitor according to claim 1, wherein
said second manipulation portion serves as a manipulation portion causing the blood pressure monitor to operate with respect to said subject selected in said first manipulation portion.
